# FASCICULE DE BREVET EUROPEEN

(11) **EP 0 530 285 B1**
(45) Date de publication et mention de la délivrance du brevet: **10.08.1994**
(21) Numéro de dépôt: 91910265.7
(22) Date de dépôt: 21.05.1991
(51) Int. Cl.: C07C 235/84, C07C 229/34, C07C 231/18, A61K 31/335

(54) **PROCEDE DE PREPARATION ENANTIOSELECTIVE DE DERIVES DE LA PHENYLISOSERINE**
VERFAHREN ZUR ENANTIOSELEKTIVEN HERSTELLUNG VON PHENYLISOSERINDERIVATEN
PROCESS FOR THE ENANTIOSELECTIVE PREPARATION OF PHENYLISOSERIN DERIVATIVES

(30) Priorité: 22.05.1990 FR 9006369
(43) Date de publication de la demande: 10.03.1993
(73) Titulaire: RHONE-POULENC RORER S.A., 92160 Antony (FR)
(72) Inventeur: CORREA, Arlène, 13560-Sao Carlos, SP (BR); DENIS, Jean-Noel Gites de Belledonne Apt. No. 3, F-38410 Uriage (FR); GREENE, Andrew-Elliot La Maison du Verger, F-38410 Uriage (FR); GRIERSON, David, Scott, F-78470 Magny-les-Hameaux (FR)
(74) Mandataire: Pilard, Jacques
(86) Numéro de dépôt international: FR9100405
(87) Numéro de publication internationale: WO9117976

(56) Documents cités:
- EP-A- 0 253 738
- EP-A- 0 336 840
- DATABASE WPIL, AN 83-31122k [13], Derwent Publications, Ltd., London, GB; & JP-A-58 029 749
- T. W. GREENE, "Protective Groups in Organic Synthesis", 1981, pages 16-22, 218-221, 261-263, Wiley-Interscience Publications, John Wiley & Sons, New York, US
- THE JOURNAL OF ORGANIC CHEMISTRY, vol. 49, no. 4, 24 février 1984, American Chemical Society, Washington, DC, US; M. MARX et al, "Reactivity-selectivity in the swern oxidation of alcohols using dimethyl sulfoxide-oxalyl chloride", pages 788-793
- A. H. HAINES, "Methods for the oxidation of organic compounds", 1985, pages 126-130, Academic Press, London, GB
- THE JOURNAL OF ORGANIC CHEMISTRY, vol. 51, no. 1, 10 janvier 1986, American Chemical Society, Washington, DC, US; J.-N. DENIS et al, "An efficient, enantioselective synthesis of the taxol side chain", pages 46-50
- JOURNAL OF THE AMERICAN CHEMICAL SOCIETY, vol. 110, no. 17, 17 août 1988, American Chemical Society, Washington, DC., US; J.-N. DENIS et al, "A highly efficient, practical approach to natural taxol", pages 5917-5919

## Description

La présente invention concerne un procédé pour la préparation énantiosélective de dérivés de la phénylisosérine de formule générale: 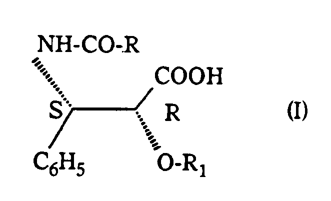
dans laquelle R représente un radical phényle ou un radical tert.butoxy et R₁ représente un groupement protecteur de la fonction hydroxy.

Dans la formule générale (I), R₁ représente plus particulièrement un radical méthoxyméthyle, éthoxy-1 éthyle, benzyloxyméthyle, (b-triméthylsilyléthoxy) méthyle, tétrahydropyrannyle ou trichloro-2,2,2 éthoxycarbonyle. De préférence, le radical R₁ est le radical éthoxy-1 éthyle.

Les produits de formule générale (I) sont utiles pour préparer les dérivés de la baccatine III et de la désacétyl-10 baccatine III de formule générale: 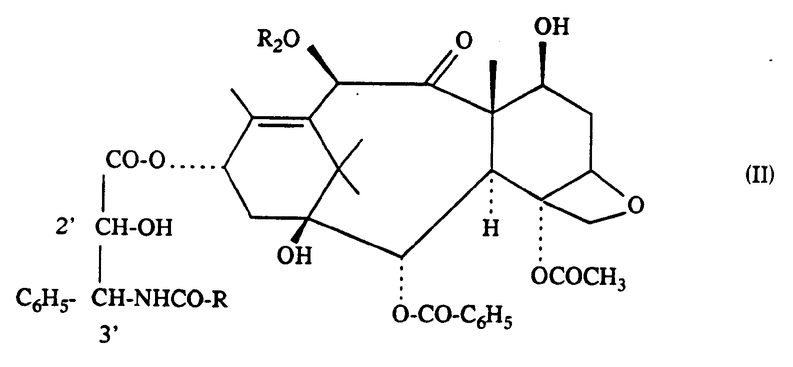
dans laquelle R représente un radical phényle ou un radical tert.butoxy et R₂ représente un atome d'hydrogène ou un radical acétyle.

Les produits de formule générale (II) dans laquelle R représente un radical phényle correspondent au taxol et au désacétyl-10 taxol et les produits de formule générale (II) dans laquelle R représente un radical tert.butoxy correspondent à ceux qui sont décrits dans le brevet européen EP 253 738.

Les produits de formule générale (II) et en particulier le produit de formule générale (II) dans laquelle R₂ représente un atome d'hydrogène et qui se présente sous la forme 2′R,3′S, présentent des propriétés antitumorales et antileucémiques particulièrement intéressantes.

Les produits de formule générale (II) peuvent être obtenus par action d'un produit de formule générale (I) sur un dérivé du taxane de formule générale: 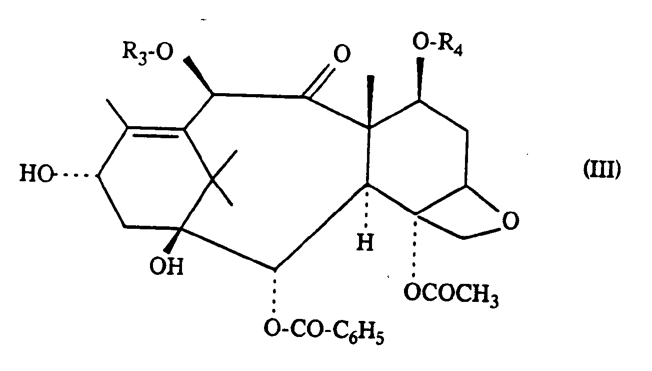
dans laquelle R₃ représente un radical acétyle ou un groupement protecteur de la fonction hydroxy et R₄ représente un groupement protecteur de la fonction hydroxy, suivie du remplacement des groupements protecteurs R₁ et R₄ et éventuellement R₃ par un atome d'hydrogène, dans les conditions décrites par J-N. DENIS et coll., J. Amer. Chem. Soc., 110 (17) 5917-5919 (1988).

Selon la présente invention, les produits de formule générale (I) sont obtenus à partir de la phénylglycine-S(+) de formule : 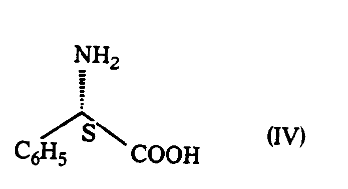
qui est traitée par un agent réducteur et par un réactif permettant d'introduire un groupement benzoyle ou t.butoxycarbonyle pour donner l'alcool de formule: 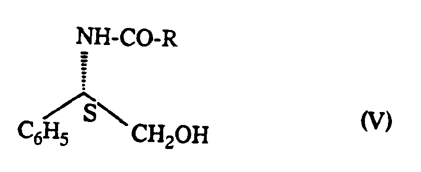
dans laquelle R représente un radical phényle ou tert.butoxy, qui est oxydé puis soumis à l'action d'un halogénure de vinylmagnésium pour donner le produit de formule: 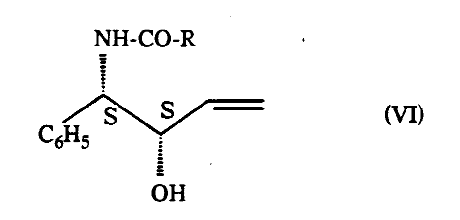
dans laquelle R représente un radical phényle ou tert.butoxy, dont la fonction hydroxy est ensuite protégée par un groupement R₁ de façon à obtenir un produit de formule générale: 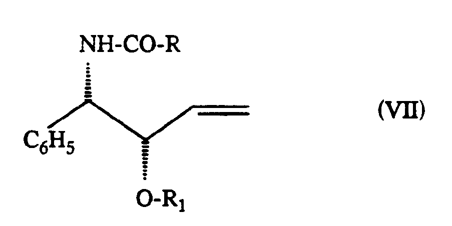
dans laquelle R représente un radical phényle ou tert.butoxy et R₁ est défini comme précédemment, qui est oxydé en produit de formule générale (I).

Selon l'invention, l'alcool de formule (V) peut être obtenu:
- soit par action d'un agent permettant d'introduire un groupement benzoyle ou t.butoxycarbonyle sur l'amino-alcool obtenu par réduction de la phénylglycine-S(+)
- soit par action d'un agent réducteur sur l'acide obtenu par action d'un agent permettant d'introduire un groupement benzoyle ou t.butoxycarbonyle sur la phénylglycine-S(+).

Quel que soit la variante du procédé utilisé, il n'est pas nécessaire d'isoler l'amino-alcool ou l'acide formé intermédiairement.

Pour la mise en oeuvre du procédé, il est particulièrement avantageux de réduire la phénylglycine-S(+) puis de faire réagir l'agent permettant d'introduire un groupement benzoyle ou tbutoxycarbonyle.

Comme agent réducteur, on utilise de préférence l'hydrure double de lithium et d'aluminium ou le borane (BH₃), de préférence sous forme de complexe avec le diméthylsulfure, en présence d'étherate de trifluorure de bore. Généralement, la réaction s'effectue dans un solvant organique inerte tel que par exemple un éther comme le tétrahydrofuranne ou le diméthoxyéthane. Généralement, la réduction s'effectue à une température comprise entre 50 et 100°C.

Comme agent permettant d'introduire un groupement benzoyle ou t.butoxycarbonyle, on utilise de préférence, selon le cas, le chlorure de benzoyle ou le dicarbonate de di-t.butyle. Généralement on opère dans un solvant organique tel que le chlorure de méthylène en présence d'une base minérale telle que la soude ou le bicarbonate ou le carbonate de sodium ou d'une base organique telle que la triéthylamine ou la diméthylamino-4 pyridine. Généralement, la réaction s'effectue entre 0°C et la température de reflux du mélange réactionnel.

Selon l'invention, l'alcool de formule (VI) est obtenu par action d'un halogénure de vinylmagnésium sur l'aldéhyde obtenu par oxydation sélective de l'alcool de formule (V).

Généralement, l'oxydation de l'alcool de formule (V) est réalisée au moyen du mélange chlorure d'oxalyle-diméthylsulfoxyde à une température inférieure à 0°C en opérant dans un solvant organique tel que le chlorure de méthylène ou le tétrahydrofuranne en présence d'une base organique telle que la triéthylamine ou la diisopropyléthylamine.

L'alcool de formule (VI) est obtenu en ajoutant l'aldéhyde à une solution d'un halogénure de vinylmagnésium, de préférence le bromure de vinylmagnésium, dans un solvant organique inerte tel que le tétrahydrofuranne éventuellement en mélange avec du chlorure de méthylène. Il n'est pas nécessaire d'isoler l'aldéhyde intermédiaire provenant de l'oxydation de l'alcool de formule (V).

En opérant de cette manière, on obtient essentiellement l'alcool de formule (VI) sous forme syn avec un excès énantiomérique supérieur à 99 %.

Le produit de formule générale (VII) peut être obtenu à partir de l'alcool de formule (VI) dans les conditions habituelles de préparation des éthers et acétals, par exemple selon les procédés décrits par J-N. DENIS et coll., J. Org. Chem., 51, 46-50 (1986).

Le produit de formule générale (I) est obtenu par oxydation de l'alcool de formule générale (VII). Il est particulièrement avantageux d'effectuer l'oxydation au moyen d'un periodate alcalin (periodate de sodium) en présence d'une quantité catalytique d'un sel de ruthénium (RuCl₃) et de bicarbonate de sodium en opérant en milieu hydro-organique tel que par exemple un mélange acétonitrile-tétrachlorure de carbone-eau. Généralement la réaction est effectuée à une température voisine de 20°C.

L'oxydation peut aussi être réalisée au moyen de permanganate de potassium, par exemple, en présence d'adogen dans un mélange pentane-eau ou en présence d'aliquat ou de dicylohexyl-18 crown-6 dans le dichlorométhane ou dans le mélange pyridine-eau. Peut aussi être utilisé le permanganate de triéthylbenzylammonium en présence de pyridine dans le dichlorométhane.

Le procédé selon l'invention permet d'obtenir diastéréosélectivement et énantiosélectivement le produit de formule générale (I) directement utilisable dans la synthèse de produits thérapeutiquement intéressants.

Les exemples suivants, donnés à titre non limitatif, montrent comment l'invention peut être mise en pratique.

### EXEMPLE 1

Dans un ballon de 500 cm3, muni d'un système d'agitation magnétique et d'un réfrigérant, on introduit, sous atmosphère d'argon, 4,55 g (120 mmoles) d'hydrure double de lithium et d'aluminium en suspension dans 210 cm3 de tétrahydrofuranne anhydre. La suspension est chauffée au reflux puis on ajoute, par petites fractions, en 15 minutes, 9,07 g (60 mmoles) de phénylglycine-S(+). On rince le réfrigérant avec 10 cm3 de tétrahydrofuranne. On chauffe au reflux pendant 6 heures.

Après refroidissement à une température voisine de 20°C, on ajoute lentement 7,28 cm3 d'une solution aqueuse de soude à 10 % (p/v) et 9,12 cm3 d'eau. On laisse réagir pendant 5 minutes puis on ajoute 14,40 g (66 mmoles) de dicarbonate de di-t.butyle en solution dans 80 cm3 de chlorure de méthylène et 200 mg (1,64 mmole) de diméthylamino-4 pyridine. On chauffe le mélange réactionnel au reflux pendant 6 heures. Après refroidissement, à une température voisine de 20°C, le mélange réactionnel hétérogène est filtré, sous pression réduite, sur sulfate de sodium anhydre. Les solides sont lavés 4 fois avec 30 cm3 de chlorure de méthylène. Les solvants sont éliminés sous pression réduite à l'évaporateur rotatif. Le résidu obtenu (15,95 g) est dissous à chaud dans 80 cm3 de chlorure de méthylène. On ajoute 350 cm3 de cyclohexane puis laisse cristalliser. Après filtration sous pression réduite, on obtient 7,503 g (31,7 mmoles) de phényl-2 t.butoxycarbonylamino-2 éthanol-S(+) sous forme de cristaux blancs.

Le résidu obtenu après concentration des eaux-mères de cristallisation est chromatographié sur une colonne de gel de silice. En éluant avec un mélange éther-hexane (1-1 en volumes), on obtient 2,986 g (12,6 mmoles) de phényl-2 t.butoxycarbonylamino-2 éthanol-S(+).

Le rendement global est de 74 %.

Le phényl-2 t.butoxycarbonylamino-2 éthanol-S(+) présente les caractéristiques suivantes:
- point de fusion : 136-137°C (après recristallisation dans le mélange chlorure de méthylène-cyclohexane)
- pouvoir rotatoire: [a]²⁴_{D} = +39,6° (c = 1,64 ; chloroforme)
- spectre infra-rouge (film) : principales bandes d'absorption à 3300, 3250, 3050, 2980, 2900, 1670, 1580, 1555, 1490, 1450, 1365, 1340, 1315, 1290, 1230, 1180, 1102, 1070, 1060, 1040, 1030, 865, 840, 760 et 700 cm⁻¹
- spectre de résonance magnétique nucléaire du proton (300 MHz ; CDCl₃ ; déplacements chimiques en ppm ; constantes de couplage J en Hz) : 1,43 (s, 9H); 1,99 (s large, 1H) ; 3,85 (d, J = 4,3, 2H); 4,77 (s large, 1H) ; 5,20 (s large, 1H); 7,26-7,38 (m, 5H)
- spectre de résonance magnétique nucléaire du ¹³C (CDCl₃) : 28,24 (3 x CH₃); 56,70 (CH) ; 66,60 (CH₂); 79,91 (C) ; 126,47 (CH) ; 127,59 (CH) ; 128,64 (CH) ; 139,63 (C) et 156,08 (C)
- spectre de masse (i.c.) (NH₃ + isobutane) : 295 (M⁺ + isobutane) ; 255 (MH⁺ + NH₃) ; 238 (MH⁺) pic de base ; 220, 206, 199, 182, 168, 150, 138, 124 et 106
- analyse élémentaire :
   calculé C 65,80 H 8,07 N 5,90
   trouvé C 65,89 H 8,02 N 5,76

### EXEMPLE 2

Dans un monocol de 100 cm3 et muni d'un système d'agitation magnétique, on introduit sous atmosphère d'argon 24 cm3 de chlorure de méthylène anhydre. On refroidit à -78°C puis on ajoute 1,05 cm3 (12 mmoles) de chlorure d'oxalyle pur. On agite la solution pendant 5 minutes à -78°C puis on ajoute en une seule fois 908 µl (12,8 mmoles) de diméthylsulfoxyde pur. La réaction est instantanée et il y a dégagement gazeux. On laisse réagir la solution pendant 5 minutes à -78°C puis on laisse remonter la température à -60°C pendant 20 minutes. On ajoute, en 15 minutes, 1,896 g (8 mmoles) de phényl-2 t.butoxycarbonylamino-2 éthanol-S(+) en solution dans 24 cm3 de chlorure de méthylène anhydre sec. On rince le ballon ayant contenu l'alcool avec 2 cm3 de chlorure de méthylène. On laisse ensuite remonter la température à -35°C en 20 minutes. On laisse réagir pendant 5 minutes à cette température puis on ajoute, en 4 minutes, 8,36 cm3 (48 mmoles) de diisopropyléthylamine pure. On laisse remonter la température en 10 minutes jusqu'à -5-0°C puis on transfère la solution homogène et jaune résultante (contenant l'aldéhyde), en 4 minutes, dans 104 cm3 d'une solution 0,5M de bromure de vinylmagnésium dans un mélange tétrahydrofuranne-chlorure de méthylène (1-1 en volumes). La réaction est exothermique et nécessite la présence d'un réfrigérant. L'addition terminée, on laisse réagir la solution homogène résultante pendant 1 heure à une température voisine de 20°C puis on ajoute successivement 8 cm3 d'éthanol et 12 cm3 d'une solution aqueuse saturée en chlorure d'ammonium. On ajoute au mélange hétérogène 100 cm3 de chlorure de méthylène et 100 cm3 d'une solution aqueuse d'acide chlorhydrique 2M pour solubiliser les solides présents. On sépare les deux phases obtenues. La phase aqueuse est extraite 3 fois avec 50 cm3 de chlorure de méthylène. Les phases organiques sont réunies et sont lavées 2 fois avec 50 cm3 d'eau et 1 fois avec 50 cm3 d'une solution aqueuse saturée en chlorure de sodium. Les phases aqueuses sont réunies puis extraites encore 2 fois avec 50 cm3 de chlorure de méthylène. Les phases organiques résultantes sont réunies puis lavées 2 fois avec 20 cm3 d'eau et 20 cm3 d'une solution aqueuse saturée en chlorure de sodium. Toutes les phases organiques sont réunies puis séchées sur sulfate de sodium anhydre. Elles sont ensuite filtrées sous pression réduite sur célite et les solvants sont éliminés à l'évaporateur rotatif. Le résidu obtenu (3,75 g) est purifié sur colonne contenant 500 cm3 de gel de silice. On élue avec un mélange éther-chlorure de méthylène (5-95 en volumes). On obtient 1,70 g (6,46 mmoles) d'un mélange des formes syn et anti du phényl-1 t.butoxycarbonylamino-1 hydroxy-2 butène-3 dans le rapport 94/6. Le rendement est de 81 %. Les deux diastéréoisomères sont séparés par chromatographie sur une colonne de gel de silice en éluant avec un mélange éther-chlorure de méthylène-hexane (5-45-50 en volumes). On obtient, avec un rendement de 62 %, 1,304 g (4,96 mmoles) de phényl-1 t.butoxycarbonylamino-1 hydroxy-2 butène-3 (1S,2S) pur dont les caractéristiques sont les suivantes:
- point de fusion : 56-57°C
- pouvoir rotatoire: [a]²⁵_{D} = +O.3° (c = 1,58, chloroforme)
- spectre infra-rouge (film) : principales bandes d'absorption à 3400, 2975, 2920, 1690, 1500, 1450, 1390, 1365, 1250, 1175, 1080, 1050, 1020, 995, 920, 755 et 700 cm⁻¹
- spectre de résonance magnétique nucléaire du proton (300 MHz ; CDCl₃ , déplacements chimiques en ppm ; constantes de couplage J en Hz) : 1,40 (s, 9H); 1,9 (s large, 1H) ; 4,38 (pst, J = 4,6 et 4,8, 1H); 4,70 (s large, 1H) ; 5,20 (dt, J = 1,4 et 10,5, 1H) ; 5,26 (s large, 1H) ; 5,34 (dt, J = 1,4 et 17,2, 1H) ; 5,86 (ddd, J = 5,4, 10,5 et 17,2, 1H); 7,24-7,37 (m, 5H)
- spectre de résonance magnétique nucléaire du ¹³C (CDCl₃) : 28,12 (3 x CH₃) ; 58,74 (CH) ; 75,33 (CH) ; 79,58 (C) ; 116,36 (CH₂); 126,69 (CH) ; 127,26 (CH) ; 128,32 (CH) ; 137,17 (CH) ; 139,96 (C) ; 155,89 (C)
- spectre de masse (i.c.) (NH₃ + isobutane) : 321 (M⁺ + isobutane) ; 281 (MH⁺ + NH₃) ; 264 (MH⁺) pic de base ; 246, 225, 208, 190, 164, 124, 106
- analyse élémentaire :
   calculé C 68,41 H 8,04 N 5,32
   trouvé C 68,15 H 7,98 N 5,34

### EXEMPLE 3

Dans un monocol de 50 cm3 mis sous atmosphère d'argon et muni d'un système d'agitation magnétique, on introduit successivement 1,045 g (3,97 mmoles) de phényl-1 t.butoxycarbonylamino-1 hydroxy-2 butène-3 (1S,2S), 20 cm3 de chlorure de méthylène anhydre, 3,8 cm3 (39,7 mmoles) d'éthylvinyléther distillé et 99 mg (0,397 mmole) de p-toluènesulfonate de pyridinium. On laisse réagir pendant 4 heures à une température voisine de 20°C. La réaction terminée, on ajoute 1 goutte de pyridine puis on dilue le mélange réactionnel dans du chlorure de méthylène. On lave la phase organique 2 fois à l'eau, 2 fois avec une solution aqueuse saturée en chlorure de sodium puis on la sèche sur sulfate de sodium anhydre. Après filtration, les solvants sont éliminés sous pression réduite à l'évaporateur rotatif. On purifie le résidu obtenu (1,545 g) sur une colonne de gel de silice en éluant avec un mélange hexane-éther (80-20 en volumes). On obtient, avec un rendement de 90 %, 1,191 g (3,56 mmoles) de phényl-1 t.butoxycarbonylamino-1 (éthoxy-1 éthoxy)-2 butène-3 (1S,2S) sous la forme de deux épimères dans le rapport 55/45.

Le phényl-1 t.butoxycarbonylamino-1 (éthoxy-1 éthoxy)-2 butène-3 (1S,2S) présente les caractéristiques suivantes:
- point de fusion : 59-65°C
- pouvoir rotatoire: [a]²⁵_{D} = +14,9° (c = 1,64, chloroforme)
- spectre infra-rouge (film) : principales bandes d'absorption à 3370, 2970, 2925, 2875, 1680, 1520, 1495, 1365, 1285, 1250, 1170, 1080, 1050, 1005, 955, 930, 890, 870, 755 et 705 cm⁻¹
- spectre de résonance magnétique nucléaire du proton (300 MHz ; CDCl₃ ; déplacements chimiques en ppm ; constantes de couplage J en Hz) : 0,9 (min) et 1,07 (maj) (2t, J = 7,0, 3H); 1,05 (min) et 1,22 (maj) (2d, J = 5,3 (min) et 5,4 (maj), 3H); 1,40 (s, 9H); 2,90-2,98 et 3,05-3,51 (m, 2H); 4,16 et 4,23 (2 psdd, J = 6,6 et 7, 1H) ; 4,31 (min) et 4,62 (maj) (2q, J = 5,3 (min) et 5,4 (maj), 1H) ; 4,71 (maj) et 4,73 (min) (2m, 1H) ; 5,22 et 5,23 (2dt, J = 1,2 et 10,5, 1H) ; 5,25 et 5,30 (2dt, J = 1,2 et 17,4, 1H); 5,37 et 5,44 (2m, 1H) ; 5,77 (min) et 5,91 (maj) (2ddd, J = 7, 10,5 et 17,4, 1H) ; 7,17-7,37 (m, 5H)
- analyse élémentaire:
   calculé C 68,03 H 8,71 N 4,18
   trouvé C 68,00 H 8,78 N 4,13

### EXEMPLE 4

Dans un ballon monocol de 50 cm3 mis sous atmosphère d'argon et muni d'un système d'agitation magnétique, on met 1,09 g (3,25 mmoles) du produit obtenu à l'exemple 3 en solution dans 6,5 cm3 d'acétonitrile. On ajoute ensuite successivement 6,5 cm3 de tétrachlorure de carbone, 9,8 cm3 d'eau distillée et, sous forte agitation, 1,774 g (21,125 mmoles) de bicarbonate de sodium. On ajoute ensuite, par petites portions, 3,824 g (17,875 mmoles) de periodate de sodium. On laisse réagir sous agitation pendant 5 minutes (dégagement gazeux) puis on ajoute en une seule fois 109 mg de chlorure de ruthénium. On laisse réagir, sous forte agitation, pendant 48 heures à une température voisine de 20°C.

On dilue le mélange réactionnel avec de l'eau de façon à obtenir un volume total de 40 cm3. La phase aqueuse basique et noire est extraite 3 fois avec 40 cm3 d'éther. La phase basique est ensuite refroidie à 0°C, puis, en présence de 120 cm3 de chlorure de méthylène et sous forte agitation, elle est traitée, goutte à goutte, par 12,9 cm3 d'une solution aqueuse d'acide chlorhydrique 2M. La phase aqueuse acide résultante est extraite 8 fois avec 120 cm3 de chlorure de méthylène. Les phases organiques sont réunies et lavées avec 3 fois 40 cm3 d'eau et 1 fois 40 cm3 d'une solution aqueuse saturée en chlorure de sodium. Elles sont séchées sur un mélange de sulfate de sodium-sulfate de magnésium (1-1) et filtrées sous pression réduite sur célite. Les solvants sont éliminés sous pression réduite jusqu'à obtention d'un volume de 5 à 8 cm3. On sèche sur tamis moléculaire 4A. On sépare la phase liquide du tamis moléculaire puis le reste de solvant est éliminé à l'évaporateur rotatif.

On obtient 940 mg (2,663 mmoles) d'acide phényl-3 t.butoxycarbonylamino-3 (éthoxy-1 éthoxy)-2 propionique (2R,3S) pur sous forme d'une huile jaune pâle. Le rendement est de 82 %.

L'acide phényl-3 t.butoxycarbonylamino-3 (éthoxy-1 éthoxy)-2 propionique (2R,3S) présente les caractéristiques suivantes:
- pouvoir rotatoire: [a]²⁵_{D} = +17,6° (c = 1,16, chloroforme)
- spectre infra-rouge (film) : principales bandes d'absorption à 3700-2200, 3060, 2980, 2930, 2850, 1720, 1660, 1602, 1590, 1500, 1450, 1400, 1370, 1280, 1250, 1170, 1080, 1050, 1030, 955, 930, 890, 700 cm⁻¹
- spectre de résonance magnétique nucléaire du proton (300 MHz ; CDCl₃ ; déplacements chimiques en ppm ; constantes de couplage J en Hz) : 0,81 et 1,04 (2t, J = 7, 3H); 1,18 et 1,20 (2d, J = 5,4, 3H); 1,42 (s, 9H); 2,60-2,88 et 3,15-3,52 (m, 2H); 4,35-4,50 et 4,65-4,80 (m, 2H); 5,29 (s large, 1H) ; 5,72 (s large, 1H) ; 7,13-7,38 (m, 5H) ; 8,52 (s large, 1H).

### EXEMPLE 5

Dans un ballon bicol de 100 cm3 surmonté d'un réfrigérant, muni d'un thermomètre et d'une agitation magnétique, on introduit, sous atmosphère d'argon, 3,605 g (23,85 mmoles) de phénylglycine-S(+) et 22,5 cm3 de diméthoxyéthane anhydre. On chauffe la suspension obtenue à 72-73°C puis on ajoute, goutte à goutte pendant 15 minutes, 3,6 cm3 (29,07 mmoles) d'étherate de trifluorure de bore distillé sur hydrure de calcium. L'addition terminée, la solution homogène jaune résultante est chauffée à 68-70°C pendant une heure. On porte ensuite ce mélange réactionnel à 77-78°C puis on ajoute lentement, en 5 minutes environ, 3,82 cm3 (38,2 mmoles) d'une solution de Me₂S.BH₃ 10M dans le tétrahydrofuranne. On laisse réagir pendant 4 heures à reflux et on refroidit le milieu réactionnel final à une température voisine de 20°C. On ajoute alors très lentement 3,6 cm3 (88,9 mmoles) de méthanol sec de façon que la température du milieu réactionnel ne dépasse pas 40°C.

On chauffe ensuite ce milieu réactionnel à reflux de façon à réduire le volume d'environ de moitié, puis on ajoute très lentement 13 cm3 d'une solution aqueuse de soude 6N (77,8 mmoles). On chauffe à 85°C pendant 30 minutes.

On refroidit ensuite à 0°C puis on ajoute 13 cm3 de chlorure de méthylène. On ajoute alors 24 cm3 d'une solution de 5,73 g (26,23 mmoles) de dicarbonate de di-t-butyle dans le chlorure de méthylène et on laisse réagir à 0°C pendant 18 heures.

On ajoute du chlorure de méthylène et de l'eau. Les phases aqueuse et organique sont séparées. La phase aqueuse est extraite 4 fois avec 30 à 40 cm3 de chlorure de méthylène. Les phases organiques sont réunies, lavées 2 fois avec 10 cm3 d'une solution aqueuse d'acide chlorhydrique 2M, 2 fois avec 20 cm3 d'eau, 1 fois avec une solution aqueuse saturée en chlorure de sodium puis sont séchées sur sulfate de sodium anhydre. Après filtration, les solvants sont éliminés sous pression réduite. Le résidu solide (5,43 g) est dissous à chaud dans le minimum de chlorure de méthylène (30 cm3) puis on ajoute à la solution résultante 76 cm3 de cyclohexane. On laisse cristalliser. Après filtration sous pression réduite, on obtient 2,686 g (11,3 mmoles) de phényl-2 t.butoxycarbonylamino-2 éthanol-S(+) sous la forme de cristaux blancs. La cristallisation du résidu des eaux-mères par le même système de solvant (minimum de CH₂Cl₂-cyclohexane) conduit encore à 1,1 g (4,6 mmoles) d'alcool sous forme de cristaux blancs. Le résidu des eaux-mères est alors purifié sur colonne de gel de silice en éluant avec un mélange éther-hexane (30/70 en volumes). On obtient ainsi 0,315 g (1,33 mmole) d'alcool.

Le rendement global est de 72 %.

Le produit obtenu est identique à celui obtenu à l'exemple 1.

### EXEMPLE 6

Dans un monocol de 500 cm3 muni d'un système d'agitation magnétique et surmonté d'un réfrigérant, on introduit, sous argon, 2,52 g (66 mmoles) d'hydrure double de lithium et d'aluminium et 120 cm3 de tétrahydrofuranne anhydre. On chauffe la suspension résultante à reflux (température extérieure 80°C) puis on ajoute, en 15 minutes, par petites portions, 5,0 g (33 mmoles) de phényl-glycine-S(+). On laisse ensuite le mélange réactionnel au reflux pendant 6 heures. On laisse refroidir à une température voisine de 20°C puis on additionne lentement 4 cm3 d'une solution aqueuse d'hydroxyde de sodium à 10 % (p/v) puis 5 cm3 d'eau. On laisse réagir pendant 5 minutes puis on ajoute encore 53 cm3 de la solution aqueuse d'hydroxyde de sodium à 10 % (p/v) puis on introduit, à 0°C, 3,3 cm3 (28 mmoles) de chlorure de benzoyle. On laisse réagir 30 minutes à une température voisine de 20°C. La réaction terminée, on dilue le mélange réactionnel dans 200 cm3 de chlorure de méthylène et on ajoute 100 cm3 d'une solution aqueuse saturée en tartrate de potassium et de sodium (sel de Rochele). Après agitation, on sépare les deux phases. La phase aqueuse est lavée 2 fois avec 50 cm3 de chlorure de méthylène. Les phases organiques réunies sont lavées 3 fois avec 20 cm3 d'eau, 1 fois avec une solution aqueuse d'acide chlorhydrique 5 % et 2 fois avec une solution aqueuse saturée en chlorure de sodium puis séchées sur sulfate de sodium anhydre. Après filtration, les solvants sont éliminés sous pression réduite. Le résidu solide obtenu est mis en solution à chaud dans 400 cm3 de chlorure de méthylène et 15 cm3 de méthanol puis on laisse cristalliser. Après filtration, on obtient, avec un rendement de 62 %, 4,9 g (20,3 mmoles) de phényl-2 benzoylamino-2 éthanol-S(-) sous la forme de cristaux blancs. Le résidu obtenu après concentration des eaux-mères (2,5 g) est chromatographié sur colonne de gel de silice. En éluant avec un mélange chlorure de méthylène-acétate d'éthyle (8-2 en volumes), on obtient 1,418 g (5,88 mmoles) de phényl-2 benzoylamino-2 éthanol-S(-). Le rendement global à partir de la phénylglycine-S(+) est de 79 %.

Le phényl-2 benzoylamino-2 éthanol-S(-) présente les caractéristiques suivantes:
- point de fusion : 179-180°C (après recristallisation dans le mélange chlorure de méthylène-cyclohexane)
- pouvoir rotatoire: [a]²⁵_{D} = -17,8° (c = 1,48, méthanol)
- spectre infra-rouge (film) : principales bandes d'absorption caractéristiques à 3300, 1630, 1600, 1580, 1520, 1310, 1290, 1265, 1120, 1075, 1040, 1030, 880, 840, 800, 750 et 700 cm⁻¹
- spectre de résonance magnétique nucléaire du proton (300 MHz ; CDCl₃ ; déplacements chimiques en ppm ; constantes de couplage J en Hz) : 2,40 (t, J = 5,5, 1H) ; 3,93 (dd, J = 4,8 et 5,5, 2H); 5,18 (dt, J = 4,8 et 6,5, 1H) ; 6,71 (s large, 1H) ; 7,16-7,42 (m, 8H); 7,70-7,73 (m, 2H)
- spectre de résonance magnétique nucléaire du ¹³C (CDCl₃ + CD₃OD) : 55,78 (CH) ; 65,29 (CH₂); 126,59 (CH) ; 127,01 (CH) ; 127,48 (CH) ; 128,40 (CH) ; 128,53 (CH); 131,58 (CH) ; 134,01 (C); 139,22 (C); 166,17 (C)
- spectre de masse (i.c.)(NH₃ + isobutane) : 242 (MH⁺) ; 224 (M⁺-OH) ; 210 (M⁺-CH₂OH) ; 122; 105 (C₆H₅CO⁺).
- analyse élémentaire:
   calculé C 74,66 H 6,27 N 5,81
   trouvé C 74,45 H 6,10 N 5,91

### EXEMPLE 7

Dans un ballon bicol de 100 cm3 mis sous atmosphère d'argon et muni d'un système d'agitation magnétique, on introduit 16 cm3 de chlorure de méthylène sec. On refroidit à -78°C puis on ajoute 1,05 cm3 (12 mmoles) de chlorure d'oxalyle pur. On laisse tourner la solution pendant 5 minutes à -78°C puis on additionne d'un coup 0,908 cm3 (12,8 mmoles) de diméthylsulfoxyde pur. La réaction est instantanée et il y a un dégagement gazeux. On laisse réagir pendant 5 minutes à -78°C puis on laisse remonter la température à -60°C en 20 minutes. On ajoute, en 15 minutes, 1,881 g (7,8 mmoles) de phényl-2 benzoylamino-2 éthanol-S(-) en suspension dans 25 cm3 d'un mélange chlorure de méthylène-diméthylsulfoxyde (24-1 en volumes). On rince le ballon ayant contenu cette suspension avec 5 cm3 de chlorure de méthylène puis on laisse remonter la température du mélange réactionnel résultant à -35°C en 20 minutes. On laisse réagir à cette température pendant 5 minutes puis on ajoute, en 4 minutes, 8,36 cm3 (48 mmoles) de diisopropyléthylamine pure. On laisse remonter la température pendant 5 minutes puis on refroidit à -78°C. On agite le mélange réactionnel homogène (contenant l'aldéhyde) pendant 5 minutes à cette température puis on le transfère dans 104 cm3 d'une solution (0,5M) de bromure de vinylmagnésium dans un mélange tétrahydrofuranne-chlorure de méthylène (1-1 en volumes). La réaction est exothermique et nécessite la présence d'un réfrigérant. L'addition terminée, on laisse réagir la solution homogène résultante 1 heure à une température voisine de 20°C puis on ajoute successivement 8 cm3 d'éthanol et 12 cm3 d'une solution aqueuse saturée en chlorure d'ammonium. On ajoute au mélange hétérogène 100 cm3 de chlorure de méthylène et 100 cm3 d'acide chlorhydrique 2M pour solubiliser les solides. On sépare les deux phases obtenues. La phase aqueuse est extraite 3 fois avec 50 cm3 de chlorure de méthylène. Les phases organiques sont réunies puis elles sont lavées 2 fois avec 20 cm3 d'eau et 1 fois avec 20 cm3 d'une solution aqueuse saturée en chlorure de sodium. Elles sont séchées sur sulfate de sodium anhydre. Après filtration, les solvants sont éliminés sous pression réduite à l'évaporateur rotatif. Le résidu obtenu (2,85 g) est chromatographié sur une colonne de gel de silice. En éluant avec un mélange chlorure de méthylène-acétate d'éthyle (95-5 en volumes), on obtient, avec un rendement de 56 %, le phényl-l benzoylamino-1 hydroxy-2 butène-3, forme syn (1,168 g, 4,37 mmoles) et avec un rendement de 13 %, le phényl-1 benzoylamino-1 hydroxy-2 butène-3, forme anti (0,28 g, 1,05 mmole). On récupère 169 mg (0,7 mmole, 9 %) de produit de départ, le phényl-2 benzoylamino-2 éthanol-S(-).

Le phényl-1 benzoylamino-1 hydroxy-2 butène-3, forme syn présente les caractéristiques suivantes:
- point de fusion : 135-136°C (après recristallisation dans le mélange chlorure de méthylène-cyclohexane)
- pouvoir rotatoire: [a]²³_{D} = -49.9° (c = 1,035, chloroforme)
- spectre infra-rouge (film) : principales bandes d'absorption caractéristiques à 3300, 1620, 1525, 1510, 1335, 1295, 1120, 1080, 995, 920 et 700 cm⁻¹
- spectre de résonance magnétique nucléaire du proton (200 MHz ; CDCl₃ ; déplacements chimiques en ppm ; constantes de couplage en Hz) : 2,40 (d, J = 3,9, 1H); 4,55 (ddd, J = 3,5, 3,5 et 5, 1H) ; 5,23 (dt, J = 1,5 et 10,5, 1H) ; 5,26 (dd, J = 3,5 et 7,6, 1H) ; 5,40 (dt, J = 1,5 et 17,1, 1H) ; 5,94 (ddd, J = 5, 10,5 et 17,1, 1H) ; 6,98 (d, J = 7,6, 1H) ; 7,24-7,54 (m, 8H); 7,80-7,83 (m, 2H)
- spectre de résonance magnétique nucléaire du ¹³C (CDCl₃) : 57,71 (CH) ; 75,31 (CH) ; 116,58 (CH₂); 126,89 (CH) ; 127,04 (CH) ; 127,68 (CH) ; 128,56 (CH) ; 128,72 (CH) ; 131,60 (CH) ; 134,31 (C); 137,41 (CH) ; 139,60 (C) ; 167,54 (C)
- spectre de masse (i.c.) (NH₃ + isobutane) : 268 (MH⁺) ; 250 (M⁺-OH) ; 210; 105 (C₆H₅CO⁺).

Le phényl-1 benzoylamino-1 hydroxy-2 butène-3, forme anti présente les caractéristiques suivantes:
- point de fusion : 176-177°C (après recristallisation dans le mélange chlorure de méthylène-cyclohexane)
- pouvoir rotatoire: [a]²³_{D} = -16,8° (c = 1,1, chloroforme)
- spectre infra-rouge (dans l'huile de vaseline) : principales bandes d'absorption caractéristiques à 3300, 1620, 1580, 1520, 1450, 1300, 1115, 1080, 1060, 1035, 985, 920, 870, 820, 800, 750 et 700 cm⁻¹
- spectre de résonance magnétique nucléaire du proton (200 MHz ; CDCl₃ ; déplacements chimiques en ppm ; constantes de couplage J en Hz) : 2,43 (s large, 1H); 4,60 (m, 1H) ; 5,23 (dt, J = 1,5 et 10,4, 1H) ; 5,33 (dd, J = 4 et 8, 1H) ; 5,34 (dt, J = 1,5 et 17, 1H) ; 5,79 (ddd, J = 5, 10,4 et 17, 1H) ; 6,88 (d, J = 8, 1H) ; 7,3-7,54 (m, 8H); 7,78-7,82 (m, 2H)
- spectre de résonance magnétique nucléaire du ¹³C (CDCl₃) : 58,23 (CH) ; 75,28 (CH) ; 117,29 (CH₂); 126,94 (CH) ; 127,52 (CH) ; 127,80 (CH) ; 128,53 (CH) ; 128,58 (CH); 131,64 (CH) ; 134,14 (C) ; 136,30 (CH) ; 137,56 (C) ; 167,23 (C)
- spectre de masse (i.c.) (NH₃ + isobutane) : 268 (MH⁺) ; 250 (M⁺-OH) ; 210; 105 (C₆H₅CO⁺).

### EXEMPLE 8

Dans un monocol de 50 cm3 mis sous atmosphère d'argon et muni d'un système d'agitation magnétique, on introduit successivement 708 mg (2,65 mmoles) de phényl-1 benzoylamino-1 hydroxy-2 butène-3, forme syn, 13,5 cm3 de chlorure de méthylène sec, 2,53 cm3 (1,911 g, 26,5 mmoles) d'éthyl vinyl éther et 66,5 mg (0,265 mmole) de p.toluènesulfonate de pyridinium (PTSP). On laisse réagir le mélange réactionnel homogène résultant pendant 4 heures à une température voisine de 20°C. La réaction terminée, on ajoute 1 goutte de pyridine puis on dilue le mélange réactionnel dans 20 cm3 de chlorure de méthylène. On lave la phase organique 1 fois avec 20 cm3 d'eau. La phase aqueuse est extraite 2 fois avec 20 cm3 de chlorure de méthylène. Les phases organiques réunies sont lavées 2 fois avec 20 cm3 d'eau et 1 fois avec 10 cm3 d'une solution aqueuse saturée en chlorure de sodium puis séchées sur sulfate de sodium anhydre. Après filtration, les solvants sont éliminés sous pression réduite à l'évaporateur rotatif. On chromatographie le résidu obtenu (992 mg) sur une colonne de gel de silice. En éluant avec un mélange hexane-éther (6-4 en volumes), on obtient le phényl-1 benzoylamino-1 (éthoxy-1 éthoxy)-2 butène-3, forme syn (818 mg, 2,41 mmoles) avec un rendement de 91 %. Le produit obtenu est sous la forme d'un mélange équimolaire des deux épimères (détermination par le spectre de résonance magnétique nucléaire du proton).

Le phényl-1 benzoylamino-1 (éthoxy-1 éthoxy)-2 butène-3, forme syn présente les caractéristiques suivantes:
- point de fusion : 85,5-87°C
- pouvoir rotatoire: [a]²²_{D} = 34,4° (c = 1,6, chloroforme)
- spectre infra-rouge (film) : principales bandes d'absorption caractéristiques à 3300, 2975, 2920, 2870, 1630, 1600, 1580, 1520, 1485, 1320, 1125, 1080, 1030, 990, 920 et 700 cm⁻¹
- spectre de résonance magnétique nucléaire du proton (200 MHz ; CDCl₃ ; déplacements chimiques en ppm ; constantes de couplage J en Hz) : 1,00 et 1,08 (2t, J = 7, 3H); 1,09 et 1,29 (2d, J = 5,3, 3H); 2,98-3,56 (m, 2H); 4,36 et 4,66 (2q, J = 5,3, 1H) ; 4,29-4,43 (m, 1H) ; 5,12-5,46 (m, 3H); 5,81 et 5,99 (2ddd, J = 6,5, 10,4 et 17,1, 1H) ; 7,05 (d, J = 8, 1H); 7,15-7,55 (m, 8H); 7,74-7,92 (m, 2H)
- spectre de masse (i.c.) (NH₃ + isobutane) : 340 (MH⁺) ; 294, 268, 250, 211, 105 (C₆H₅CO⁺).

### EXEMPLE 9

Dans un monocol de 15 cm3 mis sous atmosphère d'argon et muni d'un système d'agitation magnétique, on met 254 mg (0,75 mmole) de phényl-1 benzoylamino-1 (éthoxy-1 éthoxy)-2 butène-3, forme syn en solution dans 1,5 cm3 d'acétonitrile. On ajoute ensuite successivement 1,5 cm3 de tétrachlorure de carbone, 2,25 cm3 d'eau distillée et, sous bonne agitation, 409,5 mg (4,875 mmoles) de bicarbonate de sodium solide. On ajoute ensuite, par petites portions, 882 mg (4,125 mmoles) de periodate de sodium. On laisse réagir le milieu réactionnel hétérogène pendant 5 minutes (dégagement gazeux) puis on ajoute en une seule fois 25,4 mg (10 % en poids) de RuCl₃ (Aldrich). Sous forte agitation, on laisse réagir le mélange réactionnel hétérogène devenu noir pendant 48 heures à une température voisine de 20°C.

La réaction terminée, on dilue le mélange réactionnel avec de l'eau de façon à obtenir un volume total de 12 cm3. La phase aqueuse basique et noire est extraite 3 fois avec 20 cm3 d'éther. La phase basique est ensuite refroidie à 0°C puis, en présence de 30 cm3 de chlorure de méthylène et sous forte agitation, elle est traitée, goutte à goutte par 3 cm3 d'une solution aqueuse d'acide chlorhydrique 2M. La phase aqueuse acide résultante est extraite 8 fois avec 35 cm3 de chlorure de méthylène. Les phases organiques sont réunies et lavées avec 3 fois 8 cm3 d'eau et 1 fois 10 cm3 d'une solution aqueuse saturée en chlorure de sodium. Elles sont séchées sur un mélange sulfate de sodium-sulfate de magnésium (1-1 en poids) et filtrées sous pression réduite sur célite. Les solvants sont éliminés sous pression réduite jusqu'à concentration d'un volume de 5 à 10 cm3. On sèche sur tamis moléculaire 4Å.

On sépare la phase liquide du tamis moléculaire puis le reste de solvant est éliminé à l'évaporateur rotatif. On obtient 183 mg (0,512 mmole) d'acide phényl-3 benzoylamino-3 (éthoxy-1 éthoxy)-2 propionique pur sous forme d'un solide blanc. Le rendement est de 68 %.

Le produit obtenu est sous la forme d'un mélange équimoléculaire des deux épimères (détermination par le spectre de résonance magnétique nucléaire du proton).

L'acide phényl-3 benzoylamino-3 (éthoxy-1 éthoxy)-2 propionique présente les caractéristiques suivantes:
- point de fusion : 93-94°C
- pouvoir rotatoire: [a]²⁵_{D} = -21,2° (c = 0,69, méthanol)
- spectre infra-rouge (film) : principales bandes d'absorption caractéristiques à 3425, 3600-2100, 3060, 3025, 2975, 2925, 1740, 1640, 1600, 1580, 1520, 1480, 1440, 1300, 1140, 1075, 1020, 950, 920, 865, 800, 765 et 700 cm⁻¹
- spectre de résonance magnétique nucléaire du proton (300 MHz ; CDCl₃ ; déplacements chimiques en ppm ; constantes de couplage J en Hz) : 0,90 et 1,07 (2t, J = 7, 3H); 1,24 (d, J = 5,3, 3H); 2,88-2,99 et 3,24-3,45 (2m, 2H); 4,50 et 4,63 (2d, J = 2,4, 1H) ; 4,60 et 4,81 (2q, J = 5,3, 1H) ; 5,74-5,80 (m, 1H) ; 7,26-7,52 (m, 8H); 7,78-7,83 (m, 2H); 7,0-7,8 (s large, 1H).

## Revendications

1. Procédé de préparation énantiosélective de dérivés de la phénylisosérine de formule générale: 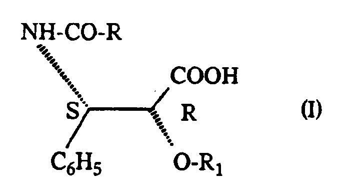 dans laquelle R représente un radical phényle ou tert.butoxy et R₁ représente un groupement protecteur de la fonction alcool, caractérisé en ce que l'on traite la phénylglycine-S(+) par un agent réducteur et un agent permettant d'introduire un groupement benzoyle ou t.butoxycarbonyle pour donner un alcool de formule : 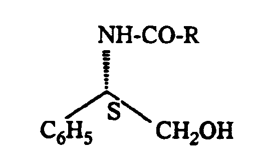 dans laquelle R est défini comme précédemment qui est oxydé puis soumis à l'action d'un halogénure de vinylmagnésium pour donner un produit de formule : 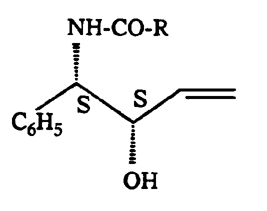 dans laquelle R est défini comme précédemment dont la fonction hydroxy est ensuite protégée par un groupement R₁ de façon à obtenir un produit de formule générale : 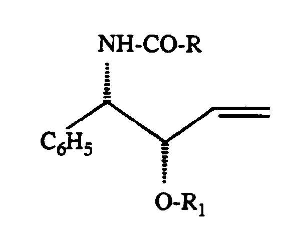 dans laquelle R et R₁ sont définis comme précédemment, qui est oxydé en produit de formule générale (I).

2. Procédé selon la revendication 1 pour la préparation d'un produit de formule générale (I) dans laquelle R₁ représente un groupement protecteur de la fonction alcool choisi parmi les radicaux méthoxyméthyle, éthoxy-1 éthyle, benzyloxyméthyle, (b-triméthylsilyléthoxy) méthyle, tétrahydropyrannyle et trichloro-2,2,2 éthoxycarbonyle.

3. Procédé selon la revendication 1 caractérisé en ce que l'on réduit la phénylglycine-S(+) au moyen d'un agent réducteur choisi parmi l'hydrure double de lithium et d'aluminium et le borane (BH₃), éventuellement sous forme de complexe avec le diméthylsulfure, puis fait agir un réactif permettant d'introduire un groupement benzoyle ou t.butoxycarbonyle sur l'amino alcool obtenu.

4. Procédé selon la revendication 1 caractérisé en ce que l'on traite la phénylglycine-S(+) par un réactif permettant d'introduire un groupement benzoyle ou t.butoxycarbonyle puis réduit l'acide obtenu au moyen d'un agent réducteur choisi parmi l'hydrure double de lithium et d'aluminium et le borane (BH₃), éventuellement sous forme de complexe avec le diméthylsulfure.

5. Procédé selon l'une des revendications 3 ou 4 caractérisé en ce que le réactif permettant d'introduire un groupement t.butoxycarbonyle est le dicarbonate de di-t.butyle.

6. Procédé selon l'une des revendications 3 ou 4 caractérisé en ce que le réactif permettant d'introduire un groupement benzoyle est le chlorure de benzoyle.

7. Procédé selon la revendication 1 caractérisé en ce que l'on oxyde le phényl-2 t.butoxycarbonylamino-2 éthanol ou le phényl-2 benzoylamino-2 éthanol au moyen d'un mélange chlorure d'oxalyle- diméthylsulfoxyde puis fait réagir l'aldéhyde intermédiairement obtenu sur un halogénure de vinylmagnésium pour obtenir le phényl-1 t.butoxycarbonylamino-1 hydroxy-2 butène-3 ou le phényl-1 benzoylamino-1 hydroxy-2 butène-3 dont on protège la fonction hydroxy selon les méthodes connues de préparation des éthers et des acétals.

8. Procédé selon la revendication 1 caractérisé en ce que l'on oxyde le phényl-1 t.butoxycarbonylamino-1 hydroxy-2 butène-3 ou le phényl-1 benzoylamino-1 hydroxy-2 butène-3 dont la fonction hydroxy est protégée, au moyen d'un periodate alcalin en présence d'une quantité catalytique d'un sel de ruthénium ou au moyen de permanganate de potassium puis isole le dérivé de la phénylisosérine de formule générale (I).

## Patentansprüche

1. Verahren zur enantioselektiven Herstellung von Phenylisoserinderivaten mit der allgemeinen Formel: 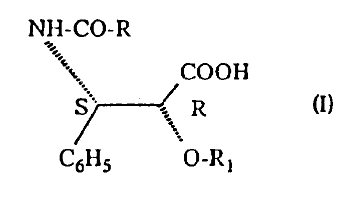 in der R einen Phenyl- oder tert-Butoxyrest darstellt und R₁ eine Schutzgruppe Für die Alkohiolfunktion, dadurch gekennzeichnet, daß man das S(+)-Phenylglycin mit einem Reduktionsmittel behandelt und mit einem Reagens zum Einbau einer Benzoyl- oder tert-Butoxygruppierung, wodurch ein Alkohol mit folgender Formel entsteht: 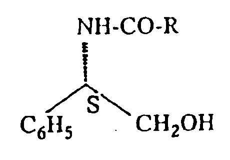 in der R wie vorher definiert ist, ein Alkohol, der oxidiert wird, auf den man dann Vinylmagnesiumchlorid einwirken läßt, wordurch ein Produkt mit folgender Formel entsteht: 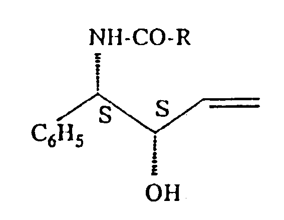 in der R wie vorher definiert ist, ein Produkt, dessen Hydroxylgruppe danach durch eine Gruppierung R₁ geschützt wird, so daß man ein Produkt mit folgender allgemeiner Formel erhält: 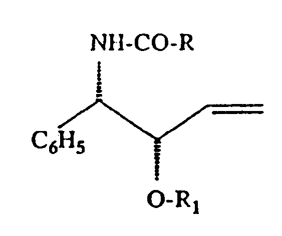 in der R und R₁ wie vorher definiert sind, ein Produkt, das zu einem Produkt mit der allgemeinen Formel (I) oxidiert wird.

2. Werfahren nach Anspruch 1 zur Herstellung eines Produkts mit der allgemeinen Formel (I), in der R₁ eine Schutzgruppe für die Alkoholfunktion darstellt, ausgewählt aus folgenden Resten: Methoxymethyl-, 1-Ethoxyethyl-, Benzyloxymethyl-, b-Trimethylsilylethoxymethyl-, Tetrahydropyranyl- und 2,2,2-Trichlorethoxycarbonylrest.

3. Verfahren nach Anspruch 1, dadurch gekennzeichnet, daß man das S(+)-Phenylglycin mit einem Reduktionsmittel reduziert, ausgewählt aus dem Lithiumaluminiumhydrid und dem Boran (BH₃), gegebenenfalls in Form eines Komplexes mit Dimethylsulfid, daß man dann ein Reagens zum Einbau einer Benzoyl- oder tert-Butylcarbonylgluppierung auf den erhaltenen Aminoalkohol einwirken läßt.

4. Verfahren nach Anspruch 1, dadurch gekennzeichnet, daß man das S(-)-Phenylglycin mit einem Reagens zum Einbau einer Benzoyl- oder tert-Butoxycarbonylgruppierung behandelt, daß man dann die erhaltene Säure mit einem Reduktionsmittel reduziert, ausgewählt aus dem Lithiumaluminiumhydrid und dem Boran (BH₃), gegebenenfalls in Form eines Komplexes mit Dimethylsulfid.

5. Verfahren nach einem der Ansprüche 3 oder 4, dadurch gekennzeichnet, daß das Reagens zum Einbau einer tert-Butoxycarbonylgruppierung das Di-tert-butyldicarbonat ist.

6. Verfahren nach einem der Ansprüche 3 oder 4, dadurch gekennzeichnet, daß das Reagens zum Einbau einer Benzoylgruppierung das Benzyolchlorid ist.

7. Verfahren nach Anspruch 1, dadurch gekennzeichnet, daß man das 2-Phenyl-2-tert-butoxycarbonylaminoethanol oder das 2-Phenyl-2-benzoylaminoethanol mit einer Mischung aus Oxalylchlorid und Dimethylsulfoxid oxidiert, daß man dann den als Zwischenprodukt erhaltenen Aldehyd mit einem Vinylmagnesium alogenid reagieren läßt, um das 1-Phenyl-1-tert-butoxycarbonylamino-2-hydroxy-3-buten oder das 1-Phenyl-1-benzoylamino-2-hydroxy-3-buten zu erhalten, dessen Hydroxylgruppe man nach den zur Herstellung von Ethern und Acetalen bekannten Methoden schützt.

8. Verfahren nach Anspruch 1, dadurch gekennzeichnet, daß man das 1-Phenyl-1-tert-butoxycarbonylamino-2-hydroxy-3-buten oder das 1-Phenyl-1-benzoylamino-2-hydroxy-3-buten, dessen Hydroxygruppe geschützt ist, mit einem Alkaliperiodat in Gegenwart einer katalytischen Menge eines Rutheniumsalzes oder mit Hilfe von Kaliumpermanganat oxidiert, daß man dann das Phenylisoserinderivat mit der allgemeinen Formel (I) isoliert.

## Claims

1. Process for the enantioselective preparation of phenylisoserine derivatives of general formula: 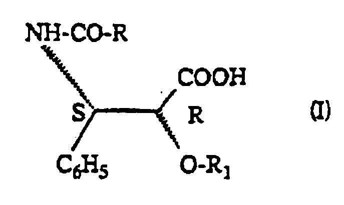 in which R represents a phenyl or tert-butoxy radical and R₁ represents a group for protecting the alcohol functional group, characterized in that S(+)-phenylglycine is treated with a reducing agent and an agent permitting the introduction of a benzoyl or tert-butoxycarbonyl group to give an alcohol of formula: 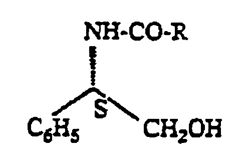 in which R is defined as above, which is oxidized and then subjected to the action of a vinylmagnesium halide to give a product of formula: 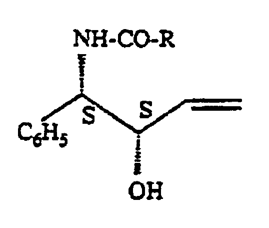 in which R is defined as above, whose hydroxy functional group is then protected with a group R₁ so as to obtain a product of general formula: 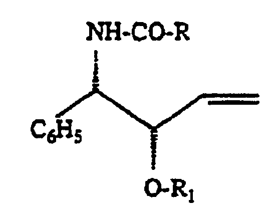 in which R and R₁ are defined as above, which is oxidized to the product of general formula (I).

2. Process according to Claim 1 for the preparation of a product of general formula (I) in which R₁ represents a group for protecting the alcohol functional group chosen from methoxymethyl, 1-ethoxyethyl, benzyloxymethyl, (b-trimethylsilylethoxy)methyl, tetrahydropyranyl and 2,2,2-trichloroethoxycarbonyl radicals.

3. Process according to Claim 1, characterized in that S(+)-phenylglycine is reduced by means of a reducing agent chosen from lithium aluminium hydride and borane (BH₃), optionally in the form of a complex with dimethyl sulphide, and then a reagent permitting the introduction of a benzoyl or t-butoxycarbonyl group is reacted with the amino alcohol obtained.

4. Process according to Claim 1, characterized in that S(+)-phenylglycine is treated with a reagent permitting the introduction of a benzoyl or t-butoxycarbonyl group and then the acid obtained is reduced by means of a reducing agent chosen from lithium aluminium hydride and borane (BH₃), optionally in the form of a complex with dimethyl sulphide.

5. Process according to one of Claims 3 or 4, characterized in that the reagent permitting the introduction of a t-butoxycarbonyl group is di-t-butyl dicarbonate.

6. Process according to one of Claims 3 or 4, characterized in that the reagent permitting the introduction of a benzoyl group is benzoyl chloride.

7. Process according to Claim 1, characterized in that 2-phenyl-2-t-butoxycarbonylaminoethanol or 2-phenyl-2-benzoylaminoethanol is oxidized by means of an oxalyl chloride-dimethyl sulphoxide mixture, and then the aldehyde obtained as intermediate is reacted with a vinylmagnesium halide to give 1-phenyl-1-t-butoxycarbonylamino-2-hydroxy-3-butene or 1-phenyl-1-benzoylamino-2-hydroxy-3-butene whose hydroxy functional group is protected according to known methods for the preparation of ethers and acetals.

8. Process according to Claim 1, characterized in that 1-phenyl-1-t-butoxycarbonylamino-2-hydroxy-3-butene or 1-phenyl-1-benzoylamino-2-hydroxy-3-butene whose hydroxy functional group is protected, is oxidized by means of an alkali metal periodate in the presence of a catalytic quantity of a ruthenium salt or by means of potassium permanganate and then the phenylisoserine derivative of general formula (I) is isolated.
